# EUROPEAN PATENT SPECIFICATION

(11) **EP 2 786 730 B1**
(45) Date of publication and mention of the grant of the patent: **15.03.2017**
(21) Application number: 13738258.6
(22) Date of filing: 18.01.2013
(51) Int. Cl.: A61F 13/15, A61F 13/472, A61F 13/534, A61F 13/539, A61F 13/475, A61F 13/537, A61F 13/53, A61F 13/47

(54) **DISPOSABLE ABSORBENT ARTICLE**
SAUGFÄHIGER EINWEGARTIKEL
ARTICLE ABSORBANT JETABLE

(30) Priority: 19.01.2012 JP 2012009450
(43) Date of publication of application: 08.10.2014
(73) Proprietor: Unicharm Corporation, Ehime 799-0111 (JP)
(72) Inventor: NAKAO, Hitomi, Kanonji-shi Kagawa 769-1602 (JP); NAKAJIMA, Kaiyo, Kanonji-shi Kagawa 769-1602 (JP)
(74) Representative: Peter, Julian
(86) International application number: PCT/JP2013/050889
(87) International publication number: WO 2013/108864

(56) References cited:
- JP-A- 2004 298 454
- JP-A- 2006 068 551
- JP-A- 2009 000 512
- JP-A- 2009 153 706
- US-A- 4 758 240
- US-A1- 2004 253 892
- US-A1- 2006 189 954

## Description

### {Technical Field}

The present invention relates to disposable absorbent articles such as sanitary napkins, incontinence pad, urine-absorbent pads and pantiliners.

### {Background}

Conventionally, disposable absorbent articles formed with a plurality of depressed spots which are concave from the upper surface side of an article in a thickness direction of the article are known. For example, the patent literature 1 discloses an absorbent article including a liquid-permeable topsheet, a liquid-impermeable backsheet and an absorbent layer lying between these sheets wherein, on the upper side surface of the article, the topsheet is formed with the dot-like depressed spots and the absorbent layer is formed with line-segment-like depressed spots in such a manner that the respective depressed spots may overlap each other in the thickness direction.

### {Citation List}

### {Patent Literature}

{PTL 1}: JP 4732495 B

Further prior art in this technical field is disclosed in US 2006/189954 A1,
US 2004/253892 A1 and US 4,758,240 A.

### {Summary}

### {Technical Problem}

According to the absorbent article disclosed in the PTL 1, an arrangement of the depressed spots of the topsheet and the depressed spots of the absorbent layer so as to overlap each other in the thickness direction certainly makes it possible to reduce a thickness of the absorbent article as a whole with respect to the rest and to assure rapid diffusion of bodily fluids in these depressed spots.

However, due to the arrangement of the depressed spots of the topsheet and the depressed spots of the absorbent layer both formed on the upper surface side of the article, there is a likelihood that stiffness of the article in these depressed spots might be increased and, in consequence, a desired flexibility of the article on the upper surface side might be decreased. Further, when the topsheet is formed with the depressed spots from the upper surface side so as to overlap the depressed spots previously formed on the upper surface side of the absorbent layer, a pressure is concentrated into these depressed spots. Consequently, there is a likelihood that the topsheet as well as the absorbent layer might be partially broken and superabsorbent polymer particles contained within the absorbent layer might fall off outward from the absorbent layer. Meanwhile, when a thickness of the absorbent layer is previously dimensioned to be thinner to avoid such disadvantageous result, a liquid-absorption capacity of the absorbent layer will be decreased and, when a depth of the depressed spots is previously dimensioned to be smaller, it will become difficult to form the absorbent article which is locally thinner.

An object of the present invention is to provide a disposable absorbent article improved so that a desired flexibility can be assured and the thickness dimension of the article can be locally reduced without decreasing the liquid-absorption capacity of the article.

### {Solution to Problem}

To solve the object set forth above, the present invention relates to a disposable absorbent article having the features of claim 1

### {Advantageous Effects of Invention}

In the absorbent article according to the present invention, at least one of a first end region and a second end region, a plurality of first depressed spots concaved from an upper surface side toward a rear surface side to an absorbent layer and a plurality of second depressed spots concaved from the rear surface side toward the upper surface side are arranged at internals so as to be spaced apart from each other in a longitudinal direction as well as in a transverse direction wherein a part of the first depressed spots and a part of the second depressed spots overlap each other in a thickness direction. Such an arrangement makes it possible to thin the end regions as a whole without sacrificing a desired flexibility.

### {Brief Description of Drawings}

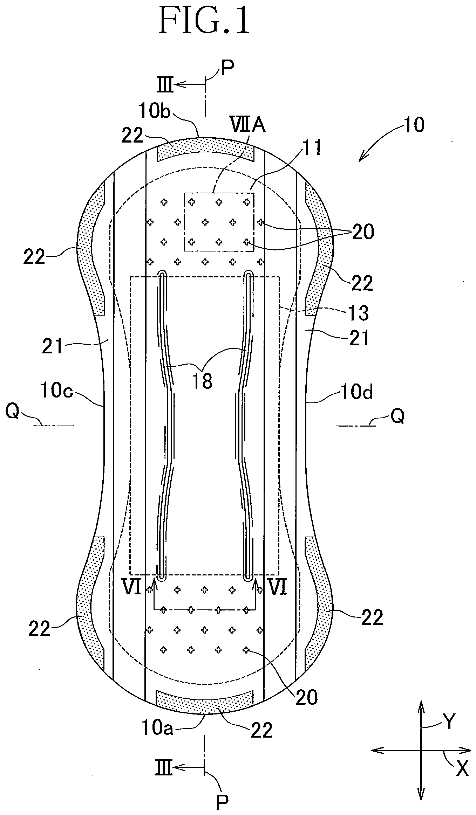

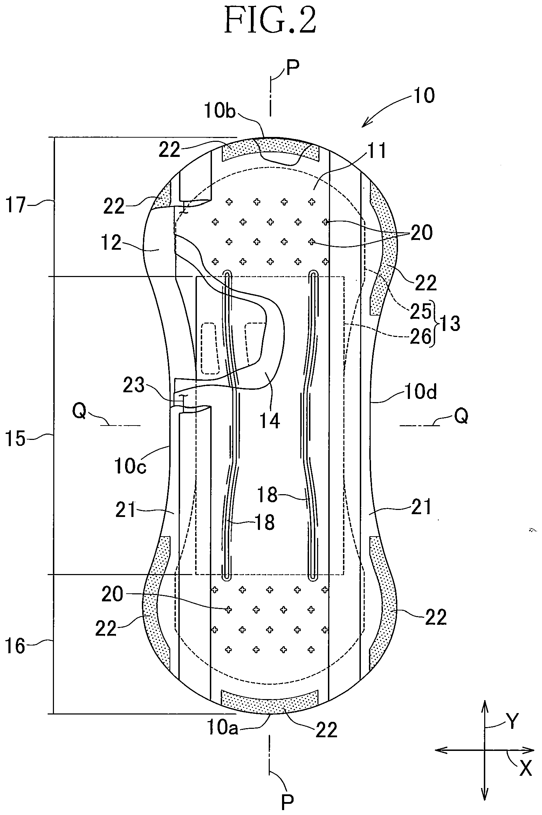

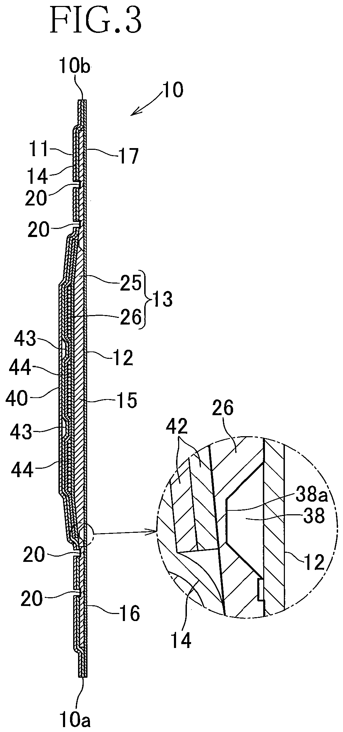

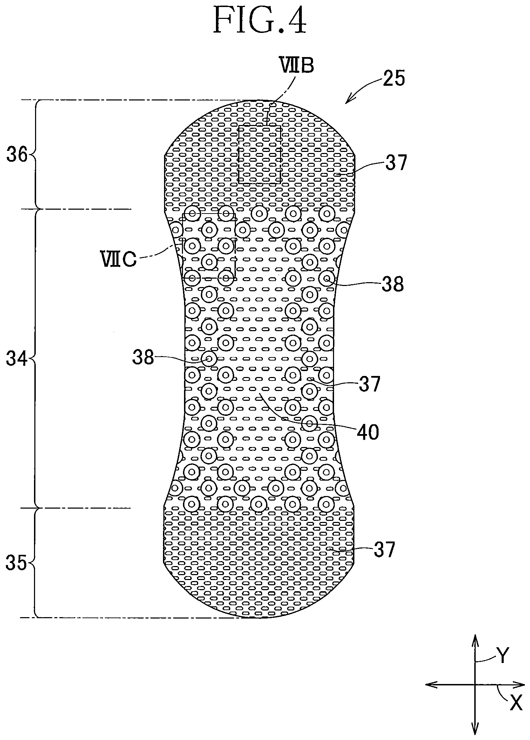

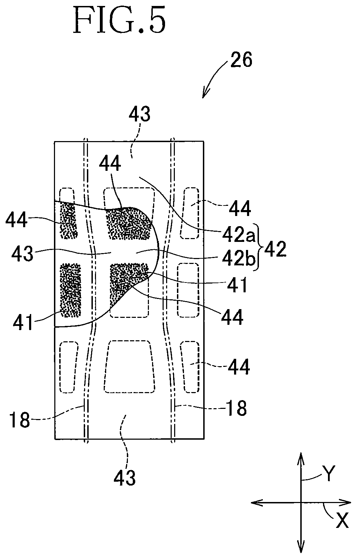

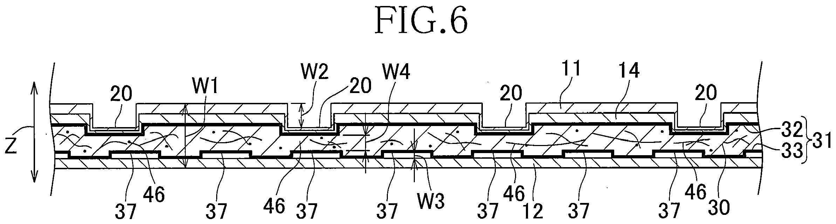

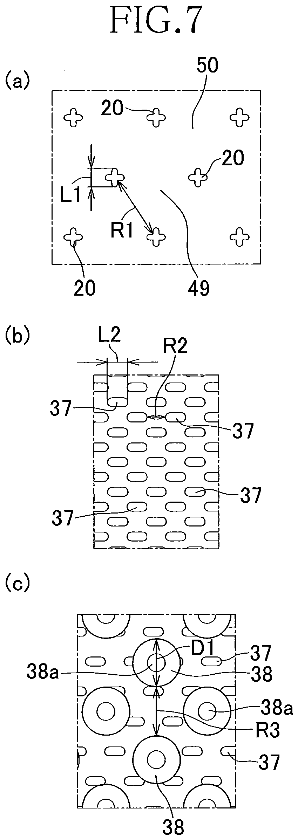

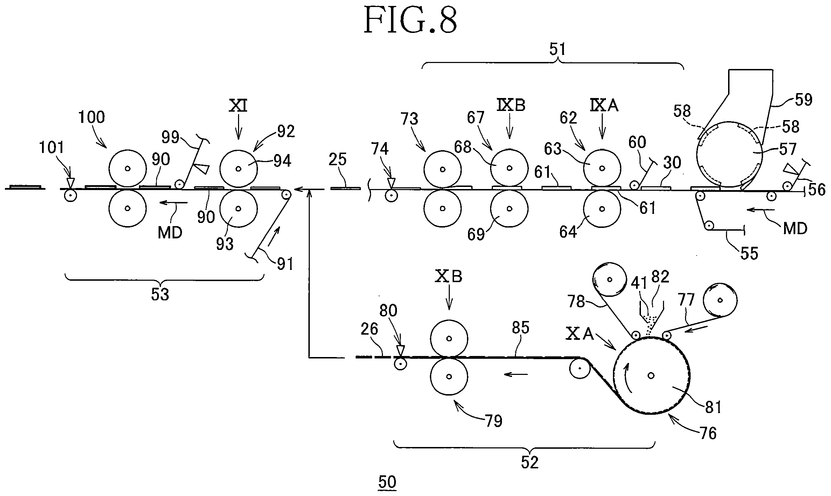

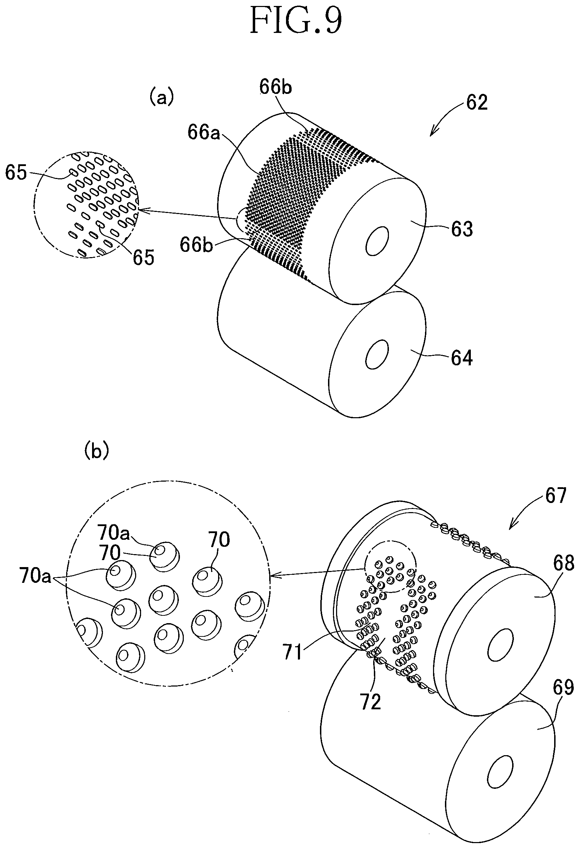

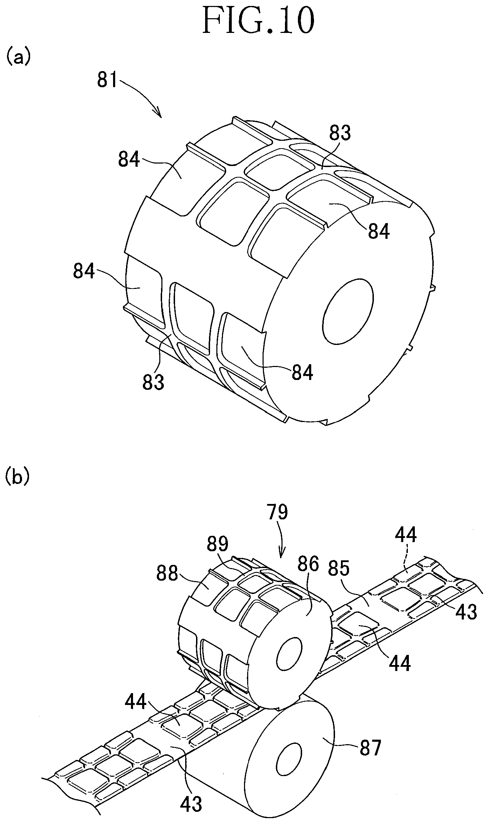

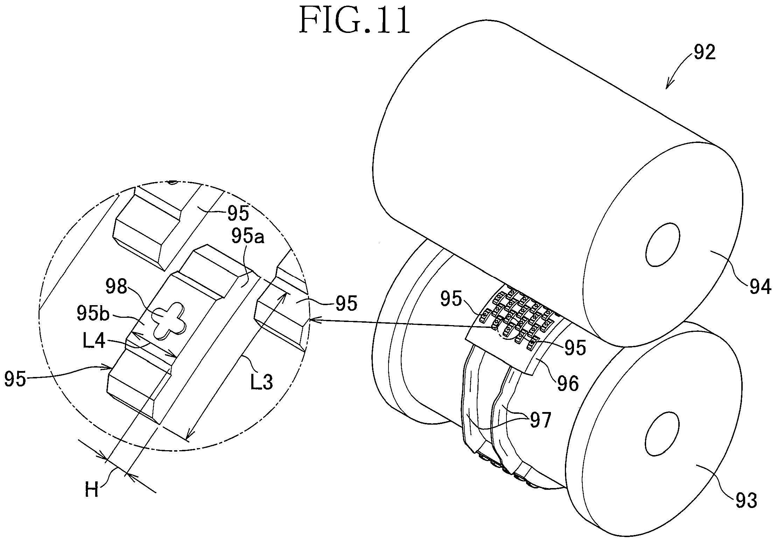

{Fig. 1} Fig. 1 is a plan view of a disposable absorbent article according to the present invention.
{Fig. 2} Fig. 2 is a partially cutaway plan view of the disposable absorbent article according to the present invention.
{Fig. 3} Fig. 3 is a schematic sectional view taken along line III-III in Fig. 1.
{Fig. 4} Fig. 4 is a plan view of a first absorbent member as viewed from the back.
{Fig. 5} Fig. 5 is a partially cutaway plan view of a second absorbent member.
{Fig. 6} Fig. 6 is a schematic sectional view taken along line VI-VI in Fig. 1.
{Fig. 7} Fig. 7(a) is a partially scale-enlarged diagram of a region enclosed by chain line VIIA, Fig. 7 (b) is a partially scale-enlarged diagram of a region enclosed by chain line VIIB and Fig. 7 (c) is a partially scale-enlarged diagram of a region enclosed by chain line VIIC.
{Fig. 8} Fig. 8 is a schematic diagram illustrating a manufacturing apparatus for the disposable absorbent article.
{Fig. 9} Fig. 9(a) is a scale-enlarged perspective view of a first embossing/debussing station of the manufacturing apparatus and Fig. 9(b) is a scale-enlarged perspective view of a second embossing/debussing station of the manufacturing apparatus.
{Fig. 10} Fig. 10(a) is a scale-enlarged perspective view of a rotary drum in a SAP-dispersing station of the manufacturing apparatus and Fig. 10(b) is a scale-enlarged perspective view of a heat-seal station of the manufacturing apparatus.
{Fig. 11} Fig. 11 is a scale-enlarged perspective view of a third embossing/debussing station of the manufacturing apparatus.

### {Description of Embodiments}

An absorbent article 10 has a longitudinal direction Y, a transverse direction X, a thickness direction Z, an upper surface (skin-contact surface), a reverse surface (non-skin-contact surface), a longitudinal axis P extending in parallel to the longitudinal direction Y and a transverse axis Q extending in parallel to the transverse direction X wherein the article 10 is formed substantially in a symmetric fashion about the longitudinal axis P as well as about the transverse axis Q.

Referring to Figs. 1 through 3, the absorbent article 10 is contoured by first and second end edges 10a, 10b, both side edges 10c, 10d concavely curved toward the longitudinal axis P and includes a liquid-permeable topsheet 11 lying on the upper surface side, a backsheet 12 lying on the reverse surface side, an liquid absorbent layer 13 lying between these both sheets 11, 12 and an intermediate sheet 14 lying between the topsheet 11 and the absorbent layer 13. For convenience of description, the article 10 is sectionalized into a central region 15, a first end region 16 extending from the central region 15 to the first end edge 10a and a second end region 17 extending from the central region 15 to the second end edge 10b.

The central region 15 is provided with a pair of compressed grooves 18 concaved from the upper surface side in the thickness direction so as to be spaced apart from each other in the transverse direction X and to extend in the longitudinal direction Y. Each of the first and second end regions 16, 17 is provided with a plurality of first depressed spots 20 concaved in the thickness direction from the upper surface side so as to be spaced apart from each other in the longitudinal direction Y as well as in the transverse direction X. Each of the first depressed spots 20 has a generally cross shape in a planar view and these first depressed spots 20 are arranged in at least one of the first and second end regions 16, 17, preferably in both end regions 16, 17 and, in any case, the first depressed spots 20 are arranged in respective sub-regions of the first and second end regions 16, 17 in which the absorbent layer 13 is present.

The topsheet 11 and the backsheet 12 extend outward from a peripheral edge of the absorbent layer 13 and are bonded to each other with the use of appropriate bonding means, for example, hot-melt adhesive around the peripheral edge of the absorbent layer 13. The backsheet 12 extends further outward in the transverse direction X beyond the topsheet 11 and a pair of side sheets 21 are bonded to the skin-contacting surface of respective protruding portions of the backsheet 12. The topsheet 11, the backsheet 12 and respective portions of the side sheets 21 constitute end flaps extending outward from the absorbent layer 13 in the longitudinal direction Y and side flaps extending outward from the absorbent layer 13 in the transverse direction X. The peripheral edges of the respective end flaps and the peripheral edges of the respective side flaps are partially sealed under hot embossing/debossing to form sealed regions 22. Overlapping regions of the respective sheets 11, 12, 14, 21 are bonded together not only under a fiber intertangling effect along the compressed grooves 18 but also by means of hot-melt adhesive (not shown).

Each of the side sheets 21 has a fixed edge portion partially forming the side flap and the end flap and a sleeve- or loop-like distal edge portion wherein at least one thread-, string- or strand-like elastic element 23 is attached under tension within the distal edge portion so as to be elastically contractible in the longitudinal direction Y. The side sheets 21 cooperate with the associated elastic elements 23 to form leakage-barrier cuffs adapted to restrict the leakage of bodily fluids out of the article 10.

The topsheet 11 may be formed from various types of liquid-permeable fibrous nonwoven fabrics, for example, air-through fibrous nonwoven fabrics, porous plastic films or laminates thereof, each having a mass per unit area in a range of about 20 g/m2 to about 40 g/m2. The backsheet 12 may be formed from liquid-impermeable and air-permeable plastic films, poorly-liquid permeable fibrous nonwoven fabrics or laminates thereof.

The intermediate sheet 14 may formed from various types of air-permeable and liquid-permeable fibrous nonwoven fabrics, for example, air-through nonwoven fabrics having a mass per unit area in a range of about 15 g/m2 to about 45 g/m2. The intermediate sheet 14 may be optionally provided to improve cushioning characteristics for the wearer's skin, to restrict diffusion of bodily fluids and to keep the topsheet 11 and the absorbent layer 13 distanced from each other, thereby preventing bodily fluids from flowing back to the topsheet 11. The side sheets 21 may be formed from impermeable SMS (spunbond/meltblown/spunbond) fibrous nonwoven fabrics, spunbond fibrous nonwoven fabrics, plastic sheets made of polyethylene or a laminate thereof, each having a mass per unit area in a range of about 10 g/m2 to about 30 g/m2.

The absorbent layer 13 is of a double-layered structure including a first absorbent member (lower layer) 25 lying on the rear surface side and a second absorbent layer (upper layer) 26 lying on the upper surface side. The first absorbent member 25 defines an outline of the absorbent layer 13 having a central portion which extends in the central region 15 toward the first and second end regions 16, 17 and is curved inward. The second absorbent member 26 has a generally rectangular shape of which a length dimension in the longitudinal direction Y is smaller than the corresponding length dimension of the first absorbent member 25. The compressed grooves 18 are arranged so as to overlap the second absorbent member 26. In the article 10, the central region 15 is of a multilayered structure including two layers. A plurality of the absorbent members may be arranged as described above to in this manner to absorb a larger amount of bodily fluids as immediately as possible.

Referring to Figs. 4 and 6, the first absorbent member 25 includes a liquid-absorbent core 30 obtained by mixing discrete and water-insoluble superabsorbent polymer particles (SAP) having water absorption capacity tenfold or more of its own mass and a mass per unit area in a range of about 100 g/m2 to 300 g/m2 with wood fluff pulp having a mass per unit area in a range of about 200 g/m2 to about 400 g/m2 and, optionally, thermoplastic synthetic fibers (staple fibers) having a mass per unit area of 30% or less and then molding the mixture into a predetermined shape and a core wrap sheet 31 adapted to wrap an entirety of the liquid-absorbent core 30, thereby improving the shape retention and liquid diffusivity of the core 30. The core wrap sheet 30 includes a permeable first sheet 32 lying on the upper surface side and a permeable or a hardly-permeable second sheet 33 lying on the rear surface side. While the first depressed spots 20 are illustrated to be concaved from the upper surface side toward the rear surface side in a manner that the topsheet 11, the intermediate sheet 14 and the absorbent layer 13 are depressed, these spots may be concaved at least from the upper surface side toward the rear surface side of the absorbent layer 13 or from the intermediate sheet 14 toward the absorbent layer 13 as long as the advantageous effects as described later of the present invention are ensured.

Referring again to Fig. 4, the first absorbent member 25 has an intermediate portion 34 lying in the central region 15, a first end portion 35 lying in the first end region 16 and a second end portion 36 lying in the second end region 17. The first end portion 35 and the second end portion 36 are approximately the same in sizes (areas) thereof and the first absorbent member 25 are provided on its entire rear surface side with a plurality of second depressed spots 37 arranged so as to be spaced apart from each other in the longitudinal direction Y as well as in the transverse direction X. The intermediate portion 34 is provided with a plurality of third depressed spots 38 concaved from the rear surface side in the thickness direction so as to be spaced apart from each other in the longitudinal direction Y as well as in the transverse direction X. In the intermediate portion 34, the second depressed spots 37 and the third depressed spots 38 partially overlap with each other.

Each of the second depressed spots 37 has a generally oval shape which is horizontally long, i.e., extends in the direction of the transverse axis Q so as to be orthogonal to the longitudinal axis P. These second depressed spots 37 are densely arranged in the first and second end portions 35, 36 and sparsely arranged in the intermediate portion 34. Referring to the locally scale-enlarged sectional view of Fig. 3, the third depressed spot 38 is approximately circular as viewed in a plan view and tapered toward the rear surface side so that a width dimension thereof is gradually reduced and centrally formed with a bottom 38a. The intermediate portion 34 is formed on the rear surface side thereof with these third depressed spots 38 except a central zone (non-depressed zone) and, in consequence, the central zone 40 is more bulky than the remaining zone of the intermediate portion 34.

Referring to Fig. 5, the second absorbent member 26 includes discrete SAP 41 and a core wrap sheet 42 adapted to wrap SAP 41. The core wrap sheet 42 includes a first sheet 42a and a second sheet 42b lying on the rear surface side. The second absorbent member 26 is defined by a sealed region 43 in which the two sheets 42a,42b are bonded to each other and absorbent regions 44 surrounded by the sealed region 43 and containing the superabsorbent polymer particles. The second absorbent member 26 contains none of wood fluff pulp and is formed of the superabsorbent polymer particles wrapped by the core wrap sheet 42. Accordingly, the second absorbent member 26 is thinner than the first absorbent member 25 and, when the first absorbent member is laminated with the second absorbent member 26 in the central region 15, there is no anxiety that the laminated portion might become unacceptably bulky and deteriorate a sense of wearing.

The absorbent region 44 of the absorbent member 26 is divided into nine sub-regions and the compressed grooves 18 (illustrated by imaginary lines in Fig. 5) are arranged so as to overlap the sealed region 43. The absorbent region 44 may be divided into a plurality of sub-regions in this manner to prevent the superabsorbent polymer particles might be unacceptably migrated within the respective liquid- absorbent sub-regions 44. The compressed grooves 18 may be arranged so as to overlap the sealed region 43 but not the absorbent sub-regions 44 to avoid a likelihood that the superabsorbent polymer particles might be crushed and the absorption capacity thereof might be deteriorated when the compressed grooves 18 are formed. A small amount of the superabsorbent polymer particles may be present in the sealed region so far as the desired effects are ensured. While a small amount of the superabsorbent polymer particles may sometimes be scattered into the sealed region 43 in the course of spraying them onto the absorbent sub-regions 44, such small amount can be tolerated so far as the sealing function is not inhibited. Further, the number of the absorbent sub-regions 44 may be freely varied, for example, divided into two or four and it is also possible to form grooves along positions overlapping the compressed grooves 18 without dividing the absorbent region into a plurality of sub-regions.

Referring to Fig. 6, the first depressed spots 20 extend in the thickness direction Z of the article 10 from the upper surface side toward the rear surface side through the topsheet 11 and the intermediate sheet 14 to the second absorbent member 26 and the first absorbent member 25 of the absorbent layer 13. In the first and second end regions 16, 17 of the article 10, a part of the first depressed spots 20 and a part of the second depressed spots 37 overlap each other in the thickness direction Z. Regarding respective dimensions of the absorbent layer 13, a dimension W1 of the first end region 16 of the absorbent layer 13 in the thickness direction Z (equal to the dimension of the second end region 17 in the thickness direction Z) is in a range of about 2.5 mm to about 4.0 mm, a dimension W2 (depth) of the first depressed spot 20 in the thickness direction X is in a range of about 1.0 mm to about 1.5 mm, a dimension W3 (depth) of the second depressed spot 37 in the thickness direction Z is in a range of about 0.2 mm to 0.5 mm and a dimension W4 of the thinnest region (most thin region) of the absorbent layer 13 in which the first depressed spots 20 and the second depressed spots 37 overlap each other in the thickness direction Z is in a range of about 1.4 mm to about 2.3 mm. In this regard, while cross-sectional shapes of the respective depressed spots 20, 37 are illustrated to be generally rectilinear in Fig. 6 which is the schematic sectional view, the respective depressed spots are formed really under compression by curved protrusions and the fibers once depressed partially restore bulkiness after the embossing/debossing work in the course of the manufacturing process. In consequence, the real cross-sectional shape of the respective depressed spots 20, 37 are not rectilinear but rather irregular.

In the absorbent layer 13 according to this embodiment, the first absorbent member 25 is provided on the rear surface side thereof with the second depressed spots 37 and there would otherwise be a likelihood that a bulk restoring force directed to the upper surface side might make the upper surface side too bulky to deteriorate a wearing sense. However, the topsheet 11, the intermediate sheet 14 and the second absorbent member 26 in a laminated state are depressed from the upper surface side to form the first depressed spots 20 and thereby it is possible to restrict a bulk restoration of the first absorbent member 25 toward the upper surface side and to ensure a desired wearing sense in the first and second end regions 16, 17. When the first and second end regions 16, 17 are depressed to a desired thickness from one of the upper surface side and the rear surface side, there is a likelihood that the surface from which the first and second end regions 16, 17 are depressed might hardened and, in consequence, flexibility might be remarkably deteriorated. However, the first and second end regions may be depressed from both surface sides to reduce the thickness without deteriorating the flexibility. When the article 10 is used as a sanitary napkin, the article is dispensed in the form individually packaged in a plastic envelope and, in many case, the sanitary napkin is packaged in a doubled up state or a state of folded in three. In the case of the absorbent article 10 according to the present invention, the first and second end regions 16, 17 is thinner than the conventional article without sacrifice of the flexibility and, even after the article has been folded for packaging so that the first end region 16, the second end regions 17 and the central region 15 may overlap each other, the article can be packaged more compactly than the conventional articles.

Referring to Fig. 4, the first absorbent member 25 is provided in entirety of the intermediate portion 34 with a plurality of the second depressed spots 37 sparsely arranged and in the zone except the central zone 40 with the third depressed spots 38 so as to be spaced apart from each other in the longitudinal direction Y as well as in the transverse direction X. With such arrangement, the central zone 40 is more bulky than the remaining zone. With such arrangement, the zone of the article 10 corresponding to the central zone 40 assures high cushioning characteristics and assures the wearer to experience soft tactual sense when the central zone 40 comes in contact with the wearer¹ urethral orifice.

The absorbent layer 13 according to the present invention may be used in various types of articles such as sanitary napkins and incontinent pads. For example, when the article is used as the adult incontinent pad targeted to aged persons from 50s to 70s, it is required to absorb bodily fluids in a range of about 100 cc to about 170 cc for every urination. Consequently, an external shape and thickness of the incontinence pad are necessarily increased and a thickness of the absorbent layer is relatively increased especially after bodily fluids have been absorbed and the external shape of the incontinence pad stands out through the clothes (especially on the ventral side and dorsal side) and an external appearance of the clothes are deteriorated. When the absorbent article according to the present invention is used as the adult incontinence pad, the article is partially depressed in the first and second end portions 35, 36 from the upper surface side and the rear surface side to obtain a relatively thin article without sacrifice of the flexibility. In this way, even after bodily fluids have been absorbed, there is no likelihood that the external shape of the incontinence pad might stand out through the clothes particularly on the ventral side and the dorsal side of the wearer and the aged person can put the pad on his or her body at ease even when the wearer steps out.

Referring to Fig. 7 (a), a dimension L1 of one side of the first depressed spots 20 in the longitudinal direction Y (a dimension in the transverse direction X also) is in a range of about 3.0 mm to about 5.0 mm, a distance dimension R1 between each pair of the adjacent first depressed spots 20 is in a range of about 8.0 mm to about 12.0 mm and an area ratio of the first absorbent member 25 to the first or second end portions 35, 36 is in a range of 1% to about 10%. The first depressed spots 20 are arranged so as to be spaced apart from each other in the longitudinal direction Y as well as in the transverse direction X and a non-depressed regions 49 are defined by sub-region surrounded by the first depressed spots 20. The absorbent material of the absorbent layer 13 surrounded by the first depressed spots 20 intensely become bulky, allowing the article to provide the wearer with elastically soft tactual sense and soft impression in appearance. The shape of the first depressed spot 20 is not limited to the cruciform as in the present embodiment but the other various well known graphics such as flower patterns or star patterns, well known shapes such as trapezoid or rhombus or optional geometric shapes may be used. In this regard, when the first depressed spot 20 has a shape which is relatively long in the longitudinal direction Y or in the transverse direction X, the fibers will be pulled in in the longitudinal direction Y or in the transverse direction X and, in consequence, gathers will be formed in the corresponding direction Y or X and there is an anxiety that bodily fluids might leak along these gathers. In view of this, the depressed spot is preferably selected to have a shape extending in two directions intersecting one another. For the reason, a pressure is dispersed into the respective sides of the intersection during the manufacturing process, preventing the fibers from being pulled in the one direction and there is no anxiety that the gathers might be formed and bodily fluids might flow along these gathers.

Referring to Fig. 7 (b), a dimension L2 of the second depressed spot 37 in the transverse direction X is in a range of about 2.0 mm to about 4.0 mm, a distance dimension R2 between each pair of the adjacent second depressed spots 37 is in a range of about 0.5 mm to about 1.0 mm and an area ratio of the first absorbent member to the first and second end portions 35, 36 is in a range of 10% to 50%, preferably in a range of 15% to 25%. The second depressed spots 37 are more densely arranged on the rear surface side of the first and second portions 35, 36 of the first absorbent member 25 than in the first depressed spots 20 on the upper surface side. The second depressed spots 37 are not arranged so as to extend continuously in a predetermined direction and spaced apart from each other in the longitudinal direction Y as well as in the transverse direction X. Consequently, there is no possibility that gathers might be formed extending in a predetermined direction between each pair of the adjacent depressed spots 37 and these spots 37 have desired flexibility. While the second depressed spots 37 may have various well known shapes, the second depressed spot 37 is preferably different from the shape of the first depressed spot 20 in size as well as in shape. When the first depressed spot 20 and the second depressed spot 37 are same in size as well as in shape, the thinnest region 46 would be formed in a relatively large area and there is a likelohood that the strength of the article 10 might lowered when the positions of these depressed spots 20 and 37 are aligned with each other in the thickness direction Z.

Referring to Fig. 7 (c), the third depressed spots 38 each have a diameter D1 in a range of about 6.0 mm to about 10.0 mm and a distance dimension R3 between each pair of the adjacent third depressed spots 38 in the longitudinal direction Y is in a range of about 7.0 mm to about 11.0 mm. An area ratio of the third depressed spots 38 as a whole to the intermediate portion 34 of the first absorbent member 25 is in a range of about 10% to about 80%, preferably in a range of 40% to 60%. The third depressed spots 38 partially overlap the second depressed spots 37.

Referring to Fig. 8, a manufacturing apparatus 50 for the article 10 includes a first shaping station 51 to shape the aforesaid first absorbent member 25, a second shaping station 52 to shape the aforesaid second absorbent member 26 and an assembling station 53 to overlap the first absorbent member 25 and the second absorbent member 26 with each other. In this manufacturing apparatus, the article 10 is manufactured in a manner that the article 10 is manufactured in a flipped state (i.e., the upper surface side is underlaid). The apparatus described hereunder in reference to Fig. 8 exemplarily illustrates the manufacturing apparatus/method for the article 10 without no intention to exclude the other manufacturing apparatus/methods having similar function.

In the first shaping station 51, the absorbent cores 30 are successively supplied and stacked from a rotary drum 57 onto first carrier sheet 56 conveyed in a machine direction MD by a conveyor belt 55. The rotary drum 57 is provided on an outer peripheral surface thereof with a plurality of depositing depressions 58 each having approximately the same shape as the first absorbent member 25 and a suction function. The depressions 58 are successively deposited with absorbent material including a mixture of wood fluff pulp and discrete superabsorbent polymer particles (hereinafter referred to as SAP) supplied from a conduit partially covering the rotary drum 57 and whereby the liquid-absorbent cores 30 each molded in the predetermined shape are obtained.

The absorbent core has been deposited, the first carrier sheet 56 is conveyed in the machine direction MD under tension given by a tension rollers. Then, second carrier sheet 60 fed from a feed roller is laminated on the first carrier sheet 56. The first and second carrier sheets 56, 60 are bonded to each other in a state holding the absorbent cores 30 therebetween to form a first absorbent web 61. Then the first absorbent web 61 is subjected to press working in a first embossing/debossing unit 62 consisting of an embossing/debossing rollers 63 and a smoothing roller 64 to form the aforesaid second depressed spots 37 arranged on the upper surface of the first absorbent web 61. Referring to Fig. 9 (a), the embossing/debossing roller 63 is provided on the outer peripheral surface with a plurality of oval protrusions 65. More specifically, the embossing/debossing roller 63 is provided on the outer peripheral surface with first pressing regions 66a in which the protrusions 65 are densely arranged and second pressing regions 66b adjacent the first pressing regions 66a, in which the protrusions 65 are sparsely arranged. A distance dimension (i.e., clearance) between these two rollers 63, 64 is in a range of 0.7 mm to 1.5 mm and these two rollers 63, 64 are preferably maintained at a temperature in a range of 80°C to 95°C to assure that the first absorbent web 61 is formed with the second depressed spots 37.

Then, the first absorbent web 61 is subjected to press working in a second embossing/debossing unit 67 consisting of an embossing/debossing roller 68 and a smoothing roller 69. Referring to Fig. 9 (b), the embossing/debossing roller 68 is formed on an outer peripheral surface thereof with press regions 71 each including a plurality of dot-like protrusions 70. In a central portion of the press region 71, a non-press sub-region in which none of the protrusions is present is defined. The first absorbent web 61 is guided to pass through between the embossing/debossing roller 68 and the smoothing roller 69 both heated at a temperature in a range of about 80°C to about 90°C under a pressing effect to form the aforesaid third depressed spots 38 on the upper surface of the first absorbent web 61. Each of the protrusions 70 has a central sub-protrusion 70a.

As illustrated in Fig. 8, after having passed through the second embossing/debossing unit 67, the first absorbent web 61 is subjected to press working at a smoothing press unit 73 including a pair of smoothing rollers in order to prevent the absorbent cores 30 from becoming shapeless. After having passed through the smoothing press unit 73, the first absorbent web 61 reaches a cutting unit 74 at which the web 61 is cut along the outer shape of the absorbent core 30 to obtain a plurality of the aforesaid first absorbent members 25. The second shaping station 52 includes a spray unit 76 for SAP, a heat sealing unit 79 and a cutting unit 80. The SAP spray unit 76 includes a rotary drum 81 and a conduit 82 adapted to feed SAP 41 to the rotary drum 81 and third carrier sheet 77 is fed from a feed roller onto an outer peripheral surface of the rotary drum 81. As illustrated in Fig. 10 (a), the rotary drum 81 is provided on an outer peripheral surface thereof with a plurality of depositing depressions 84 surrounded by lattice-shaped frames 83, respectively, and having suction function. After the third carrier sheet 77 has been supplied onto the rotary drum 81, SAP 41 is sprayed from the supply tube 82 into the depositing depressions 84. After SAP 41 have been sprayed to the third carrier sheet 77, fourth carrier sheet 78 is fed onto the rotary drum 81 and laminated on the third carrier sheet 77 to form second absorbent web 85. A plurality of the depositing depressions 84 are arranged in the circumferential direction as well as in the direction orthogonal thereto. Between the two carrier sheets 77, 78, a plurality of the aforesaid absorbent regions 44 sprayed with SAP 41 and the aforesaid sealed regions 43 approximately sprayed with none of SAP 41 are formed.

Then the second absorbent web 85 is conveyed into a heat seal unit 79 consisting of a heating roller 86 and a smoothing roller 87. The heating roller 86 is provided on an outer peripheral surface with a lattice-like frame 89 and a plurality of depressions 88 similar to those provided on the outer peripheral surface of the rotary drum 81. The regions 43 are subjected to heat press working of the frame 89 and thereby sealed. After having passed through the heat seal unit 79, the second absorbent web 85 is cut into the rectangular shape at the cutting unit 80 to form the aforesaid plurality of second absorbent regions 26. While not illustrated in Fig. 8, materials for the topsheet 11, the intermediate sheet 14 and the side sheet 21 are supplied to the second absorbent member 26 shaped at the second shaping station 52.

Now a laminate the laminate materials for the aforesaid second absorbent member 26, topsheet 11, intermediate sheet 14 and side sheets are converged together with the first absorbent member 25 so as to place the first absorbent member 25 on the laminate to form a composite web 90. The composite web 90 is put on a conveyor belt 91 and conveyed in the machine direction MD and the lower surface of the composite web 90 is subjected to embossing/debossing by an embossing/debossing unit 92 consisting of an embossing/debossing roller 93 and a smoothing roller 94. Referring to Fig. 11, the embossing/debossing roller 93 is provided on a central zone of an outer peripheral surface with a plate 96 having a plurality of protrusions 95 and a pair of rib-like protrusions 97 circumferentially extending from the plate 96. As illustrated in the scale-enlarged Fig. 11, the protrusions 95 include a base 95a and sub-protrusions 95b each further including a central crucial protrusion 98. In the protrusion 95, a length dimension L3 of a long side is about 6.0 mm, a length dimension L4 of a short side is about 4.0 mm and a distance (height of the protrusion 95) H from a bottom of the base to the protrusion 98 is about 1.0 to about 1.5 mm. The composite web 90 is subjected to press working in the third embossing/debossing unit 92 so that the bottom surface thereof may be heated under a pressure by a plurality of protrusions 97 to form the first depressed spots 20 and heated by a plurality of the rib-like protrusions 97 under a pressure to form the compressed grooves 18. The two rollers 93, 94 are opposed to each other at a distance of s about 0.2 mm to about 0.06 mm, the embossing/debossing roller 93 is heated at a temperature in a range of 130°C to 160°C (temperature of the pins) and the smoothing roller 94 is not heated. During such embossing/depbossing process, if the composite web is heated under a pressure with use protrusions of a predetermined height, there will be an anxiety that the material might be locally broken. However, such anxiety can be avoided by adjusting the distance between the two rollers 93, 94 so that the lower surface of the composite web 90 may be compressed by all of the sub-protrusions 95b of the respective protrusions 95 and thereby a pressure may be appropriately distributed. As will be apparent from the plan views and the sectional views of the absorbent article according to the present inventions, the first depressed spots 20 have cross-like figures, respectively. While the composite web is compressed by the protrusions 98 and the sub-protrusions 95b in the course of the embossing/debossing work and, as an inevitable consequence, two-stage spots are formed, the portion of the composite web compressed by the sub-protrusions 95 restore a desired bulkiness.

After having passed through the third embossing/debossing unit 92, the composite web 90 is supplied with fifth carrier sheet 99 as material for the backsheet 12 and heat sealed in a seal unit 100 to form a plurality of the sealed regions 22. Finally, the composite web 90 is cut along the sealed regions 22 to form a plurality of the articles 10.

The constituent members of the article 10 are not limited to those described in the specification but the other various types of material widely used in the relevant technical field may be used without limitation unless otherwise stated. Terms "first", "second" and "third" used in the specification and claims of the present invention are used merely to distinguish the similar elements, similar positions or the other similar means.

The disclosure as described above may be summarized at least as follows:
A disposable absorbent article having a longitudinal direction, a transverse direction, a thickness direction, an upper surface side and a rear surface side, and including a permeable topsheet lying on the upper surface side, an impermeable backsheet lying on the rear surface side and an absorbent layer lying between these sheets, wherein: the article has a first end region, a second end region spaced apart from and opposed to the first end region in the longitudinal direction and a central region extending between the first end region and the second region; a plurality of first depressed spots concaved from the upper surface side toward the rear surface side in at least one of the first end region and the second end region so as to depress the absorbent layer and a plurality of second depressed spots concaved from the rear surface side to the upper surface side of the absorbent layer are arranged at internals in the longitudinal direction as well as in the transverse direction; and the first depressed spots and the second depressed spots are partially opposed to each other.

The present invention disclosed above in paragraph [0040] may include at least embodiments as follow:
(1) The first depressed spots and the second depressed spots are different from each other in shapes thereof.
(2) The absorbent layer includes an intermediate portion lying in the central region, a first end portion lying in the first end region and a second end portion lying in the second end region, and an area ratio of the first depressed spots to the first or second end portions is smaller than an area ratio of the second depressed spots to the first or second end portion.
(3) The second depressed spots are arranged on an entirety of the absorbent layer and the second depressed spots are arranged in the first and second end portions more densely than in the intermediate portion.
(4) The first depressed spots are respectively provided approximately in cross shapes.
(5) Each of the second depressed spots has oval shape longer in the transverse direction.
(6) The central region is provided on the rear surface side with a plurality of third depressed spots and the third depressed spots overlap a part of the second depressed spots in the intermediate portion.
(7) The central zone of the intermediate portion on the rear surface side defines a non-depressed zone surrounded by the third depressed spots.

### {Reference Signs List}

- 10: absorbent article
- 11: topsheet
- 12: backsheet
- 13: absorbent layer
- 15: central region
- 16: first end region
- 17: second end region
- 20: first depressed spots
- 25: first absorbent member
- 26: second absorbent member
- 34: intermediate portion
- 35: first end portion
- 36: second end portion
- 37: second depressed spots
- 38: third depressed spots
- 43: sealed region
- 44: absorbent regions
- X: transverse direction
- Y: longitudinal direction
- 34: intermediate portion
- 35: first end portion
- 36: second end portion
- 37: second depressed spots
- 38: third depressed spots
- 43: sealed region
- 44: absorbent regions
- X: transverse direction
- Y: longitudinal direction

## Claims

1. A disposable absorbent article (10) having a longitudinal direction (Y), a transverse direction (X), a thickness direction (Z), an upper surface side and a rear surface side, and including a liquid-permeable topsheet (11) lying on the upper surface side, an impermeable backsheet (12) lying on the rear surface side, an absorbent layer (13) lying between these sheets (11, 12), and an intermediate sheet (14) lying between the topsheet (11) and the absorbent layer (13); wherein:
the article has a first end region (16), a second end region (17) spaced apart from and opposed to the first end region (16) in the longitudinal direction and a central region (15) extending between the first end region (16) and the second end region (17);
a plurality of first depressed spots (20) concaved from the upper surface side toward the rear surface side in at least one of the first end region (16) and the second end region (17) in a manner that the topsheet (11), the intermediate sheet (14) and the absorbent layer (13) are depressed and a plurality of second depressed spots (37) concaved from the rear surface side to the upper surface side of the absorbent layer (13) are arranged at intervals in the longitudinal direction as well as in the transverse direction;
the absorbent layer (13) is of a double-layered structure including a first absorbent member (25) lying on the rear surface side and a second absorbent member (26) lying on the upper surface side,
the first absorbent member (25) includes a liquid-absorbent core (30) comprising a mixture of superabsorbent polymer particles (SAP) having water absorption capacity tenfold or more of its own mass and a mass per unit area in a range of 100g/m² to 300g/m² with wood fluff pulp having a mass per unit area in a range of 200g/m² to about 400g/m² and, optionally, thermoplastic synthetic fibers (staple fibers) having a mass per unit area of 30% or less,
the second absorbent member (26) contains none of wood fluff pulp and includes discrete superabsorbent polymer particles (SAP) (41) and a core wrap sheet (42), the superabsorbent polymer particles (SAP) being wrapped by the core wrap sheet (42),
the second absorbent member (26) has a generally rectangular shape of which a length dimension in the longitudinal direction Y is smaller than the corresponding length dimension of the first absorbent member (25),
the second absorbent member (26) is laminated with the first absorbent member (25) in the central region (15),
the first depressed spots (20) extend in the thickness direction of the article (10) from the upper surface side toward the rear surface side through the topsheet (11) and the intermediate sheet (14) to the second absorbent member (26) and the first absorbent member (25), and
in the first and second end regions (16, 17), a part of the first depressed spots (20) and a part of the second depressed spots (37) overlap each other in the thickness direction Z.

2. The article according to claim 1, wherein the first depressed spots (20) sand the second depressed spots (37) are different from each other in shapes thereof.

3. The article according to claim 1 or 2, wherein the absorbent layer (13) includes an intermediate portion (34) lying in the central region (15), a first end portion (35) lying in the first end region (16) and a second end portion (36) lying in the second end region (17), and an area ratio of the first depressed spots (20) to the first or second end portions (35, 36) is smaller than an area ratio of the second depressed spots (37) to the first or second end portion (36).

4. The article according to any one of claims 1 through 3, wherein the second depressed spots (37) are arranged on an entirety of the absorbent layer (13) and the second depressed spots (37) are arranged in the first and second end portions (16, 36) more densely than in the intermediate portion (34).

5. The article according to any one of claims 1 through 4, wherein the first depressed spots (20) are respectively provided substantially in cross shapes.

6. The article according to any one of claims 1 through 5, wherein each of the second depressed spots (37) has oval shape longer in the transverse direction.

7. The article according to any one of claims 3 through 6, wherein the central region (15) is provided on the rear surface side with a plurality of third depressed spots (38) and the third depressed spots (38) overlap a part of the second depressed spots (37) in the intermediate portion (34).

8. The article according to any one of claims 3 through 7, wherein the central zone of the intermediate portion (34) on the rear surface side defines a non-depressed zone surrounded by the third depressed spots (38).

## Patentansprüche

1. Wegwerfbarer absorbierender Artikel (10) mit einer Längsrichtung (Y), einer Querrichtung (X), einer Dickenrichtung (Z), einer oberen Oberflächenseite und einer hinteren Oberflächenseite und umfassend eine flüssigkeitsdurchlässige Oberlage (11), die auf der oberen Oberflächenseite liegt, ein undurchlässige Rücklage (12), die auf der hinteren Oberflächenseite liegt, eine Absorptionsschicht (13), die zwischen diesen Lagen (11, 12) liegt, und eine dazwischenliegende Lage (14), die zwischen der Oberlage (11) und der Absorptionsschicht (13) liegt; wobei:
der Artikel eine erste Endregion (16), eine zweite Endregion (17), die zu der ersten Endregion (16) beabstandet ist und ihr in die Längsrichtung gegenüberliegt, und eine Zentralregion (15), die sich zwischen der ersten Endregion (16) und der zweiten Endregion (17) erstreckt;
eine Vielzahl von ersten eingesenkten Stellen (20) konkav von der oberen Oberflächenseite Richtung der hinteren Oberflächenseite in zumindest einer der ersten Endregion (16) und der zweiten Endregion (17) in einer Weise gebildet ist, sodass die Oberlage (11), die dazwischenliegende Lage (14) und die Absorptionsschicht (13) eingesenkt sind und eine Vielzahl von zweiten eingesenkten Stellen (37) konkav ausgebildet von der hinteren Oberflächenseite zu der oberen Oberflächenseite der Absorptionsschicht (13) in Intervallen in die Längsrichtung und in die Querrichtung angeordnet sind;
die Absorptionsschicht (13) aus einer doppelschichtigen Struktur umfassend ein erstes Absorptionselement (25), das auf der hinteren Oberflächenseite liegt, und ein zweitens Absorptionselement (26), das auf der oberen Oberflächenseite liegt, gebildet ist,
das erste Absorptionselement (25) einen flüssigkeitsabsorbierenden Kern (30) umfasst, der eine Mischung aus superabsorbierenden Polymerpartikeln (SAP), die eine Wasserabsorptionskapazität zehnmal oder mehr so groß wie seine eigene Masse und eine Masse pro Flächeneinheit in einem Bereich von 100g/m² bis 300g/m² besitzt, mit Holzflockenzellstoff, der eine Masse pro Flächeneinheit in einem Bereich von 200g/m² bis ungefähr 400g/m² hat, und optional mit thermoplastischen, synthetischen Fasern (Kurzfasern) mit einer Masse pro Flächeneinheit von 30% oder weniger umfasst,
das zweite Absorptionselement (26) keinen Holzflockenstoff umfasst und diskrete superabsorbierende Polymerpartikel (SAP) (41) und eine Kernwickellage (42) umfasst, wobei die superabsorbierenden Polymerpartikel (SAP) von der Kernwickellage (42) umwickelt sind,
das zweite Absorptionselement (26) eine grundsätzlich rechteckige Form hat, von der eine Längendimension in die Längsrichtung Y kleiner ist als die korrespondierende Längendimension des ersten Absorptionselements (25),
das zweite Absorptionselement (26) mit dem ersten Absorptionselement (25) in der Zentralregion (15) laminiert ist,
sich die ersten eingesenkten Stellen (20) in die Dickenrichtung des Artikels (10) von der oberen Oberflächenseite in Richtung der hinteren Oberflächenseite durch die Oberlage (11) und die dazwischenliegende Lage (14) zu dem zweiten Absorptionselement (26) und dem ersten Absorptionselement (25) erstrecken, und
in der ersten und zweiten Endregion (16, 17) ein Teil der ersten eingesenkten Stellen (20) und ein Teil der zweiten eingesenkten Stellen (37) einander in die Dickenrichtung Z überlappen.

2. Artikel nach Anspruch 1, wobei sich die ersten eingesenkten Stellen (20) und die zweiten eingesenkten Stellen (37) in ihrer Form unterscheiden.

3. Artikel nach Anspruch 1 oder 2, wobei die Absorptionsschicht (13) einen dazwischenliegenden Abschnitt (34), der in der Zentralregion (15) liegt, einen ersten Endabschnitt (35), der in der ersten Endregion (16) liegt, und einen zweiten Endabschnitt (36), der in der zweiten Endregion (17) liegt, umfasst und ein Flächenverhältnis der ersten eingesenkten Stellen (20) zu den ersten oder zweiten Endabschnitten (35, 36) kleiner ist als ein Flächenverhältnis der zweiten eingesenkten Stellen (37) zu den ersten oder zweiten Endabschnitten (36).

4. Artikel nach Anspruch 1 bis 3, wobei die zweiten eingesenkten Stellen (37) auf einer Gesamtheit der Absorptionsschicht (13) angeordnet sind und die zweiten eingesenkten Stellen (37) in den ersten und zweiten Endabschnitten (16, 36) dichter angeordnet sind als in dem dazwischenliegenden Abschnitt (34).

5. Artikel nach einem der Ansprüche 1 bis 4, wobei die ersten eingesenkten Stellen (20) jeweils im Wesentlichen kreuzförmig vorgesehen sind.

6. Artikel nach einem der Ansprüche 1 bis 5, wobei jede der zweiten eingesenkten Stellen (37) eine ovale Form, die in die Querrichtung länger ist, hat.

7. Artikel nach einem der Ansprüche 4 bis 6, wobei die Zentralregion (15) auf der hinteren Oberflächenseite mit einer Vielzahl von dritten eingesenkten Stellen (38) vorgesehen ist und die dritten eingesenkten Stellen (38) einen Teil der zweiten eingesenkten Stellen (37) in dem dazwischenliegenden Abschnitt (34) überlappen.

8. Artikel nach einem der Ansprüche 3 bis 7, wobei die Zentralzone des dazwischenliegenden Abschnitts (34) auf der hinteren Oberflächenseite eine nicht-eingesenkte Zone bestimmt, die von den dritten eingesenkten Stellen (38) umgeben ist.

## Revendications

1. Article absorbant jetable (10) ayant une direction longitudinale (Y), une direction transversale (X), une direction d'épaisseur (Z), un côté de surface supérieure et un côté de surface arrière, et incluant une feuille supérieure perméable aux liquides (11) reposant sur le côté de surface supérieure, une feuille arrière imperméable (12) reposant sur le côté de surface arrière, et une couche absorbante (13) reposant entre ces feuilles (11, 12), et une feuille intermédiaire (14) reposant entre la feuille supérieure (11) et la couche absorbante (13) ; dans lequel :
l'article comporte une première région d'extrémité (16), une seconde région d'extrémité (17) espacée de la première région d'extrémité (16) et opposée à celle-ci dans la direction longitudinale et une région centrale (15) s'étendant entre la première région d'extrémité (16) et la seconde région d'extrémité (17) ;
une pluralité de premiers points enfoncés (20) pénétrant depuis le côté de surface supérieure vers le côté de surface arrière dans au moins une parmi la première région d'extrémité (16) et la seconde région d'extrémité (17) d'une manière selon laquelle la feuille supérieure (11), la feuille intermédiaire (14) et la couche absorbante (13) sont enfoncées et une pluralité de deuxièmes points enfoncés (37) pénétrant depuis le côté de surface arrière vers le côté de surface supérieure de la couche absorbante (13) sont agencés à intervalles dans la direction longitudinale ainsi que dans la direction transversale ;
la couche absorbante (13) est d'une structure à double couche incluant un premier élément absorbant (25) reposant sur le côté de surface arrière et un second élément absorbant (26) reposant sur le côté de surface supérieure,
le premier élément absorbant (25) inclut un coeur d'absorption de liquide (30) comprenant un mélange de particules polymères super-absorbantes (SAP) ayant une capacité d'absorption d'eau faisant dix fois ou plus sa propre masse et une masse par unité de surface dans une plage de 100 g/m² à 300 g/m² avec de la pâte de fibres de bois ayant une masse par unité de surface dans une plage de 200 g/m² à environ 400 g/m² et, éventuellement, des fibres synthétiques thermoplastiques (fibres discontinues) ayant une masse par unité de surface de 30 % ou moins,
le second élément absorbant (26) contient aucune pâte de fibres de bois et inclut des particules polymères super-absorbantes (SAP) distinctes (41) et une feuille d'enveloppement de noyau (42), les particules polymères super-absorbantes (SAP) étant enveloppées par la feuille d'enveloppement de noyau (42),
le second élément absorbant (26) a une forme généralement rectangulaire dont une dimension de longueur dans la direction longitudinale Y est inférieure à la dimension de longueur correspondante du premier élément absorbant (25),
le second élément absorbant (26) est stratifié avec le premier élément absorbant (25) dans la région centrale (15),
les premiers points enfoncés (20) s'étendent dans la direction d'épaisseur de l'article (10) depuis le côté de surface supérieure vers le côté de surface inférieure à travers la feuille supérieure (11) et la feuille intermédiaire (14) jusqu'au second élément absorbant (26) et le premier élément absorbant (25), et
dans les première et seconde régions d'extrémité (16, 17), une partie des premiers points enfoncés (20) et une partie des deuxièmes points enfoncés (37) se chevauchent dans la direction d'épaisseur Z.

2. Article selon la revendication 1, dans lequel les premiers points enfoncés (20) et les deuxièmes points enfoncés (37) sont différents les uns des autres en termes de formes de ceux-ci.

3. Article selon la revendication 1 ou 2, dans lequel la couche absorbante (13) inclut une partie intermédiaire (34) reposant dans la région centrale (15), une première partie d'extrémité (35) reposant dans la première région d'extrémité (16) et une seconde partie d'extrémité (36) reposant dans la seconde région d'extrémité (17), et un rapport de surface des premiers points enfoncés (20) sur la première ou seconde partie d'extrémité (35, 36) est inférieur à un rapport de surface des deuxièmes points enfoncés (37) sur la première ou seconde partie d'extrémité (36).

4. Article selon l'une quelconque des revendications 1 à 3, dans lequel les deuxièmes points enfoncés (37) sont agencés sur une totalité de la couche absorbante (13) et les deuxièmes points enfoncés (37) sont agencés dans les première et seconde parties d'extrémité (16, 36) de manière plus dense que dans la partie intermédiaire (34).

5. Article selon l'une quelconque des revendications 1 à 4, dans lequel les premiers points enfoncés (20) sont respectivement fournis sensiblement en sections transversales.

6. Article selon l'une quelconque des revendications 1 à 5, dans lequel chacun des deuxièmes points enfoncés (37) a une forme ovale plus longue dans la direction transversale.

7. Article selon l'une quelconque des revendications 3 à 6, dans lequel la région centrale (15) est pourvue, sur le côté de surface arrière, d'une pluralité de troisièmes points enfoncés (38) et les troisièmes points enfoncés (38) chevauchent une partie des deuxièmes points enfoncés (37) dans la partie intermédiaire (34).

8. Article selon l'une quelconque des revendications 3 à 7, dans lequel la zone centrale de la partie intermédiaire (34) sur le côté de surface arrière définit une zone non enfoncée entourée par les troisièmes points enfoncés (38).
